# EUROPEAN PATENT APPLICATION

(11) **EP 1 249 702 A1**
(43) Date of publication of application: **16.10.2002**
(21) Application number: 01900784.8
(22) Date of filing: 16.01.2001
(51) Int. Cl.: G01N 33/53, G01N 33/543, G01N 33/566

(54) **INTEGRATED SUPPORT, INTEGRATED MICRO-CONTAINER AND PERMEABLE MEMBRANE, AND METHOD FOR PRODUCTION THEREOF AND USE THEREOF**

(30) Priority: 17.01.2000 JP 2000007763
(71) Applicant: Unitec Co., Ltd., Matsudo-shi, Chiba 270-0025 (JP)
(72) Inventor: TAJIMA, Hideji, Matsudo-shi Chiba 270-0025 (JP)
(74) Representative: Bohnenberger, Johannes, Dr.
(86) International application number: JP0100223
(87) International publication number: WO01053831

(57) **Abstract**

The present invention relates to an integrated support, integrated minute vessels and a permeable membrane, and a method of making and using the same, and has an object of providing an integrated support, integrated minute vessels and permeable membrane which can be manufactured simply, cheaply, and in large quantities; which can be suitably applied to various processes, and in which the level of integration is high, the operating efficiency is high, and wherein the volumes and sizes of the support, the vessels and the membrane are suitable for handling minute quantities of liquids; and for which processing can be conducted surely and with good reliability; as well as methods of making and using the same.

The invention is constructed comprising at least one thread shaped, string shaped, tape shaped, or rod shaped long and slender base member, and a variety of substances for detection of predetermined chemical structure which are fixed side by side along the length of the base member, and the base member is rolled, laminated or arranged using a predetermined method, to give integration, and the fixed location on the layer surface of the integrated base member, of each substance for detection corresponds to the chemical structure thereof.

## Description

### BACKGROUND OF THE INVENTION

### Field of the Invention

The present invention relates to an integrated support, integrated minute vessels and a permeable membrane, and a method of making and using the same. The invention relates to all manner of fields which require the handling of minute quantities of liquids; including the fields of engineering, agricultural science incorporating foodstuffs, agricultural production and fish processing, pharmaceuticals, the medical field incorporating hygiene, health, immunity, disease and heredity, and scientific fields such as chemistry and biology.

The present invention relates to fields involving the handling of biopolymers such as genes, immune systems, and proteins, and particularly relates to integrated supports, integrated minute vessels and permeable membranes, and a method of making and using the same, which are suitable for the analysis of genes, including mutational analysis, polymorphic analysis, mapping, base sequence analysis, and mechanism analysis.

### Description of the Related Art

Currently, determinations of gene base sequences use a DNA chip wherein an oligonucleotide with a predetermined base sequence is attached to a substrate made of a semiconductor at a predetermined location.

DNA chips require the preparation of a known oligonucleotide array, which is a flat surface comprising a semiconductor film or a slide glass, on to which is spotted a minute quantity of suspensions of a plurality of different, known oligonucleotides, with the oligonucleotides fixed in an array pattern sequence. Furthermore, not only in the field of DNA chips, but in all cases where liquids formed of minute quantities of a plurality of different materials are handled, a plurality of vessels corresponding with the number of different materials are required. Moreover in handling such minute quantities of liquid, permeable membranes of sizes which correspond with the minute quantities of liquid are also required.

However in order to form a plurality of oligonucleotides on the restricted surface area of a DNA chip, it is necessary for a human operator to use a pipette apparatus and then dispense spot by spot a minute quantity of each oligonucleotide suspension onto the surface leaving a predetermined separation between adjacent spots. Unfortunately this process suffers from requiring a stressful operation to ensure the prevention of mixing between the oligonucleotide suspensions.

Because the manufacture of each DNA chip takes considerable time and effort, the manufacture of large quantities of DNA chips requires an enormous expenditure of time and labor. Particularly in the determination of various base sequences, it is necessary to be able to supply DNA chips simply, at low cost, and in large quantities.

Furthermore, and not just limited to the field of DNA chips, when handling minute quantities of a plurality of different liquids, a number of vessels which corresponds with the number of different liquids need to be used in order to avoid cross contamination. If the vessels are comparatively large with respect to the minute quantities of liquid being handled in the vessels, then the work area required is large and the operating efficiency deteriorates. However the manufacture of vessels which correspond in size with minute quantities of material is difficult and requires a considerable manufacturing effort.

Moreover, analysis of DNA chips requires the reliable measurement of a high density of light emissions generated from within a very small region.

The present invention aims to resolve the problems outlined above, with a first object of providing an integrated support, integrated minute vessels and a permeable membrane which can be manufactured simply, cheaply, and in large quantities, as well as a method of making and using the same.

A second object is to provide an integrated support, integrated minute vessels and a permeable membrane and a method of making and using the same, which can be suitably applied to various processes, and in which the level of integration is high, the operating efficiency is high, and wherein the volumes and sizes of the support, vessels and membrane are suitable for handling minute quantities of liquids.

A third object is to provide an integrated support, integrated minute vessels and a permeable membrane and a method of making and using the same, wherein the handling of minute quantities of liquids can be performed with good levels of reliability.

A fourth object is to provide an integrated support, integrated minute vessels and a permeable membrane and a method of making and using the same, wherein materials such as substances for detection such as oligonucleotides can be simply and reliably attached and stored, the encounter rate is good, and the reaction efficiency is high.

A fifth object is to provide an integrated support, integrated minute vessels and a permeable membrane and a method of making and using the same, wherein by interlocking a dispensing device and a process which utilizes the dispensing device, processing is able to be conducted simply and rapidly, with minimal reliance on the processing ability of an operator, and with human conducted processing reduced to a minimum.

A sixth object is to provide an integrated support, integrated minute vessels and a permeable membrane and a method of making and using the same, which are suitable for the handling of biopolymers such as genetic material such as DNA, immunity materials, proteins, amino acids and sugars.

### SUMMARY OF THE INVENTION

In order to resolve the above problems a first aspect of the invention is an integrated support comprising: at least one thread shaped, string shaped, tape shaped, or rod shaped long and slender base member, and a variety of substances for detection of predetermined chemical structure which are fixed side by side along the length of the base member, and the base member is rolled, laminated or arranged to give integration, so that a fixed location of each substance for detection corresponds to the chemical structure thereof. In this description a substance for detection refers to a substance which should be detected in order to either determine the unknown structure of a target substance, or to conduct various other analyses, and includes genetic matter such as oligonucleotides, biopolymers such as proteins, amino acids and sugars, microorganisms such as bacteria and viruses, and living bodies such as cells. The term chemical structure refers to, for example, a base sequence in those cases where the substance for detection is a genetic material.

Genetic material includes nucleic acids (polynucleotides), and substances such as oligonucleotides and nucleotides produced by decomposition of nucleic acids. Nucleotides are compounds produced by the glycoside bonding of the purine base of adenine or guanine, or alternatively the pyrimidine base of cytosine, thymine or urasil with the reduced form of a sugar, and the sugar portion creates phosphoric acid and an ester. Thus polynucleotides generated by polymerization of purine nucleotides and pyrimidine nucleotides are called nucleic adds. Furthermore, the substance in which the sugar portion is D-2-deoxyribose is called a deoxyribonucleotide, and is a structural component of DNA. The substance in which the sugar portion is ribose is called a ribonucleotide, and is a structural component of RNA.

A base member is formed of either a flexible material or a non-flexible material. Suitable materials include organic materials such as polyethylene, polystyrene, polypropylene, and urethane, inorganic materials such as glass fiber, ceramics, and metals, as well as organic/inorganic mixed materials such as fine grains of a ceramic laid on the surface of a tape type or film type organic material.

The aforementioned "correspondence" should preferably be achieved by relating to the position on the layer surface (integrated surface) formed from the layered structure generated by integrating the base member by rolling, lamination or arrangement. The layer surface is not necessarily a flat planar surface, and may also be an irregular surface, or a coiled or curved surface.

Furthermore, suitable techniques for integration include flat rolling, laminating or arranging to produce a flat plane, or alternatively cylindrical or prismatic rolling, laminating or arranging to form a cylinder, a prism, a circular cone, or a pyramid, as well as rolling, laminating or arranging in the manner outlined below in the third aspect of the invention.

The aforementioned "fixed location" is not limited solely to the case where the location is provided on the layer surface of the base member, and may also be provided on the surface where the base members contacts with itself, or is spaced apart, or sandwiches an auxiliary member (the side portions of the base member or the non-integrated surface). Provision on the side portion or non-integrated surface enables relatively simple large volume production, and is moreover very reliable, and as such is particularly preferable from both a manufacturing and a quality viewpoint. Furthermore, it is preferable that the fixed locations are lined up so as not to mutually contact one another.

The positioning of substances for detection with a dispensing device following integration of the base member, by dispensing and then fixing each substance in the correct location while avoiding cross contamination between each of the locations, is extremely difficult due to the closeness of adjacent fixed locations. In contrast, with the present invention the substances for detection such as oligonucleotides are bound in close formation to the expanded (unfolded) base member (a first integration), and can then be compacted together even more by rolling, laminating or arranging to give further integration (a second integration). Consequently highly integrated supports for substances for detection are able to be formed. Furthermore, even if the degree of one dimensional compactness in the first integration is not particularly high, the existence of the second integration enables a two dimensional high level of compactness to be achieved.

As described above, with the first aspect of the present invention the aforementioned integrated support is integrated by rolling, laminating or arranging a long and slender base member which is initially in an expanded state.

Consequently, the positioning and fixing of substances for detection to the base member can be carried out before integration while the base member is still in an expanded state. As a result, the positioning and fixation of the substances for detection can be conducted simply, quickly and reliably, meaning the integrated support (including DNA integrated supports) can be formed both simply and quickly.

Even if, for example, the positioning and fixation of the substances for detection on to the base member is not conducted at a one-dimensionally high level of compactness or integration, by integrating the base member a two-dimensional high degree of compactness or integration is achievable. By carrying out the integration in this manner, with two separate stages (a first integration and a second integration), the integration can be completed simply, quickly, reliably, and moreover at low cost.

If, for example, the positioning and fixation of the substances for detection onto the base member is able to be carried out at a one-dimensionally high level of compactness or integration, then the subsequent two-dimensional integration of the base member will enable an even higher degree of compactness and integration to be achieved.

Furthermore by enabling, where necessary, selection of either the expanded state or the integrated state, operations can be conducted in the most suitable state for each situation. Consequently the speed, efficiency, ease, and moreover the reliability of operations can be improved. For example, the fixation and measurements of each substance for detection at each predetermined location can be conducted in the expanded state, whereas by performing the storage, reaction processing, and measurements in the integrated state, the detection process can be made simpler, quicker, more efficient and more automated, and detections of a high degree of reliability can be achieved.

A second aspect of the present invention is an integrated support according to the first aspect of the invention, wherein the base member is provided with cavity sections comprising a channel either with or without a bottom, an aperture, or a capillary, or holding sections of a porous material, a foam material, a fibrous material, a material with an irregular surface, or an impregnating material, and moreover wherein the substances for detection are fixed to the cavity sections or holding sections.

From a manufacturing and a quality point of view, the cavity sections or holding sections should preferably be provided on the side portions of the base member.

The term "cavity section", in addition to channels, apertures, and capillaries, also incorporates the formation of concave and convex portions on the side portions or top portion, for example. Moreover, the term "holding section" refers to materials which are able to absorb or retain liquids such as various porous materials, foam materials, fibrous materials, materials with irregular surfaces, and impregnating materials, and is not limited to flexible materials such as paper, cloth, thread and cord, but also includes organic materials such as polyethylene, polystyrene, polypropylene, and urethane, and inorganic materials such as glass, ceramics, and metals which have been formed as a porous material, a foam material, a fibrous material, a material with an irregular surface, or an impregnating material.

The term "material with an irregular surface" refers to materials which appear at first glance to be smooth, but which have a plurality of microscopic irregularities or cilia formed on the side portions or top portion thereof, and includes materials such as an organic film or tape on which has been spread fine particles of a ceramic. The aforementioned cavity sections or holding sections can also be provided side by side along the length of the base member.

With this second aspect of the present invention, because the substances for detection are fixed to a cavity section or holding section provided on the base member, the fixation surface area at each fixed location is increased, meaning the amount of the substance for detection being fixed can be increased, thereby simplifying the fixation process.

Furthermore, in those cases where, for example, the cavity section is an open ended channel or a bottomless aperture, a liquid is able to pass through the cavity section, meaning the contact efficiency of the substance for detection within the liquid is improved, enabling the reaction process to be accelerated.

In those cases where each cavity section is a channel with ends, or an aperture with a bottom, in order to ensure that movement of the liquid in the direction of a normal line relative to the substrate of the support is not inhibited due to the fact that liquid cannot pass through the cavity section, the contact efficiency of the liquid with respect to the cavity section can be increased by providing on the base member a bottomless aperture or an open ended channel.

With the second aspect of the present invention, substances for detection are not supported simply on a flat substrate, but rather are supported by the cavity sections or the holding sections formed on the substrate.

As a result, in the case where a substrate of identical size with a conventional substrate supports an identical number of different types of substances for detection in an identical arrangement, the amount of each substance for detection supported by each cavity section or holding section can be increased. Consequently, when reaction processing is carried out, the detection efficiency is increased due to an expansion of the encounter rate between substances, enabling an increase in detection speed. Furthermore for measurement purposes, because the amount of light emission will also increase, measurements can be conducted simply and reliably.

Furthermore, in those cases where the amount of each substance for detection is identical with that used in conventional techniques, this aspect of the invention enables a decrease in the surface area of the layer surface necessary to support such an amount in comparison with a flat substrate. Consequently, the required surface area of the substrate surface can be reduced and the integration density increased, while smaller substrates can be manufactured.

If the overall surface area of the substrate is identical with that used in conventional techniques, then the spacing between adjacent cavity sections or holding sections can be increased, making handling easier, and enabling more reliable detection to be conducted. Furthermore, an additional plurality of different substances for detection could also be supported on a single substrate.

Moreover, during bonding of the substances for detection, since the cross-sectional area occupied by each cavity section or holding section on the substrate does not need to be as large as the area occupied in the case of bonding to a flat substrate, the bonding density can be increased.

A third aspect of the invention is an integrated support according to either one of the first or second aspects of the invention, wherein the base member is rolled, laminated or arranged in such a way that either enables or prevents expansion while bringing side portions thereof into contact with each other or while maintaining a spacing or while sandwiching an auxiliary member.

In those cases where the base member is integrated by bringing into contact the side portions thereof, even if the side portions are smooth surfaces, in those cases where there are minute irregularities seen microscopically, the substances for detection can be fixed to those irregular portions. With this third aspect of the invention, in addition to the effects observed for the first aspect, the base member is able to be integrated using a variety of methods, meaning a variety of processes can be conducted in accordance with the reactions, cleaning, efficiency and effects being sought.

A fourth aspect of the invention is an integrated support according to any one of the first through third aspects of the invention, wherein markings are attached to the base member for identifying the chemical structure of the substances for detection, or the locations on the integrated support of the substances for detection. The substances for detection can also be coded to enable identification. Furthermore, the markings can be carried out with light emitting materials, or by identification by coloring predetermined regions of the base member in different colors.

With the fourth aspect of the invention, by marking the layer surface of the integrated support, the relative location, and consequently the content of each cavity section can be assigned easily, meaning highly reliable analyses can be conducted.

A fifth aspect of the invention is an integrated support according to any one of the first through fourth aspects of the invention, further comprising a binding section for binding the base member and/or an auxiliary member in such a way that is either releasable or non-releasable. Here " binding section" is a section which houses and thus binds the integrated base member, or which utilizes an adhesive portion. By binding the base member to a prescribed standard size and generating a cartridge, handling is simplified and costs can also be reduced.

With the fifth aspect of the invention, by providing the binding section for binding the base member in such a way that is either releasable or non-releasable, the substrate can be integrated to either enable or prevent expansion. Furthermore, rigidity can also be given to the structure of the integrated support. In those cases where integration is carried out to enable expansion, the same effects as those described for the first aspect of the invention are achievable.

A sixth aspect of the invention is an integrated support according to the fifth aspect of the invention, wherein the binding section is an adhesive portion for bonding side portions of the base member and/or auxiliary member in a manner which is either releasable or non-releasable.

The term "bonding" incorporates bonding with an adhesive and physical bonding using a velcro for example.

With the sixth aspect of the invention, by providing an adhesive portion as the binding section the base member can be bound simply and reliably.

A seventh aspect of the invention is an integrated support according to any one of the first through sixth aspects of the invention, wherein a linear homoiothermal member is embedded inside the base member and/or an auxiliary member for heating or cooling purposes.

The heating or cooling linear homoiothermal member may be electrical heating wires, or a fluid passage through which a heating or cooling medium is passed. Furthermore, the homoiothermal member should preferably be provided along the length of the cavity member and/or the auxiliary member.

With the seventh aspect of the invention, by embedding a linear homoiothermal member for example inside the base member for heating or cooling purposes, heating or cooling can be carried out simply and efficiently, and moreover an integrated support is achieved which is compact and easy to handle.

An eighth aspect of the invention is a DNA integrated support comprising: a substantially flat substrate wherein at least one thread shaped, string shaped, tape shaped, or rod shaped long and slender base member is rolled, laminated or arranged in such a way that either enables or prevents expansion while bringing side portions thereof into contact with each other or while maintaining a spacing or while sandwiching an auxiliary member, to give integration; and genetic substances such as oligonucleotides which are fixed in locations along the length of the base member, either in a plurality of cavity sections which comprise channels, apertures or capillaries either with or without bottoms, or alternatively in a plurality of holding sections comprising a porous material, a foam material, a fibrous material, a material with an irregular surface, or an impregnating material, with the cavity sections or holding sections being provided along the length of the base member, and the fixed locations of each of the genetic substance correspond with base sequences for that substance. In the description above "flat substrate" includes both disc shaped and square shaped substrates. This aspect of the invention displays the same effects as those described for the first aspect.

A ninth aspect of the invention is an integrated minute vessel comprising: at least one thread shaped, cord shaped, tape shaped or rod shaped long and slender base member; and either cavity sections which comprise channels, apertures or capillaries with bottoms or ends, or holding sections formed from a porous material, a foam material, a fibrous material, a material with an irregular surface, or an impregnating material, provided on the base member, and wherein the base member is rolled, laminated or arranged to give integration. The cavity sections or holding sections may be provided side by side along the length of the base member.

With the ninth aspect of the invention an integrated and compact integrated minute vessel can be provided which is suitable for handling minute quantities of liquids. Consequently, reaction processing can be conducted simultaneously on a plurality of minute vessels, rapidly and efficiently. Furthermore in those cases where the system is expandable, the storage and removal of liquids from each of the cavity sections or holding sections can be carried out with ease.

For example, even if the positioning of the cavity sections or holding sections on the base member is not carried out at a high density or high level of integration in the first dimension, by integrating the base member a high level of compactness or integration is achieved in the second dimension. By separating the integration in this manner, into a two step process, the integration can be carried out simply, rapidly and reliably, and moreover at low cost.

If the positioning of the cavity sections or holding sections on the base member is able to be carried out at a high density or high level of integration in the first dimension, then when the base member is further integrated two dimensionally, even higher levels of compactness and integration can be achieved.

A tenth aspect of the invention is an integrated minute vessel according to the ninth aspect of the invention, wherein the base member is rolled, laminated or arranged in such a way that either enables or prevents expansion, while bringing side portions thereof into contact with each other or while sandwiching an auxiliary member, to give integration and formed into a substantially flat sheet.

With the tenth aspect of the invention, integrated minute vessels can be formed with ease. Furthermore when the base members are expanded, liquids can be stored in, and removed from each cavity section simply, rapidly, and reliably.

An eleventh aspect of the invention is an integrated minute vessel according to either one of the ninth and tenth aspects of the invention, wherein marking is provided on the layer surface of the integrated minute vessel to identify position on a layer surface.

With the eleventh aspect of the invention, coordinating each cavity section or holding section with the content stored therein is simple, and analyses can be conducted simply and reliably.

A twelfth aspect of the invention is an integrated minute vessel according to any one of the ninth through eleventh aspects of the invention, wherein the integrated minute vessel is provided with a binding section for binding the base members and/or auxiliary member in such a way that is either releasable or non-releasable.

As described above the binding section may be either a section which houses and thus binds the base member, or a section which comprises an adhesive portion, as described below in a thirteenth aspect of the invention. In those cases where the base member is housed and thus bound, by prescribing a standard size and generating a cartridge, handling is simplified and costs can also be reduced.

With the twelfth aspect of the invention, by providing the binding section for binding the base member in such a way that is either releasable or non-releasable, the substrate can be integrated to either enable or prevent expansion. Furthermore, rigidity can also be given to the structure of the integrated minute vessel.

A thirteenth aspect of the invention is an integrated minute vessel according to any one of the ninth through twelfth aspects of the invention, wherein the binding section is an adhesive portion which bonds side portions of the base member and/or the auxiliary member in such a way that these are either releasable or non-releasable.

With the thirteenth aspect of the invention by providing an adhesive portion as the binding section, the base member can be bound simply and reliably.

A fourteenth aspect of the invention is an integrated minute vessel according to any one of the ninth through thirteenth aspects of the invention, wherein the thread shaped, cord shaped, tape shaped or rod shaped base member is provided with channels with ends, or apertures with bottoms or capillaries, and/or bottomless apertures or capillaries, or open ended channels, along the direction of a normal line of the layer surface.

Bottomless apertures or open ended channels enable displacement of liquids in the direction of the normal line of the layer surface, and are used to improve the efficiency of the contact of the liquids with the channels with ends or apertures with bottoms.

With the fourteenth aspect of the invention, in addition to the channels with ends and the apertures with bottoms provided across the thickness of the base member, open ended channels or bottomless apertures are also provided as is required. As a result, the penetration of liquids through the substrate becomes possible, and the contact efficiency of the liquid with the cavity sections improves, enabling the reaction efficiency to be increased.

A fifteenth aspect of the invention is an integrated minute vessel according to any one of the ninth through fourteenth aspects of the invention, wherein a linear homoiothermal member is provided inside the base member or the auxiliary member for heating or cooling purposes.

With the fifteenth aspect of the invention, by embedding a linear homoiothermal member inside the base member for heating or cooling purposes, an integrated minute vessel can be provided which is compact and easy to handle, and for which heating or cooling can be carried out efficiently.

A sixteenth aspect of the invention is a permeable membrane comprising at least one thread shaped, string shaped, tape shaped, or rod shaped long and slender base member; and cavity sections such as penetrating channels, apertures or capillaries, or holding sections formed from a porous material, a foam material, a fibrous material, a material with an irregular surface, or an impregnating material, provided on the base member, and wherein the base member is rolled, laminated or arranged to give integration. The cavity sections or holding sections may also be provided side by side along the base member.

The sixteenth aspect of the invention provides a permeable membrane in which a penetrating plurality of cavity sections or holding sections are integrated, and moreover the cavity sections are either expandable or non-expandable. In those cases where expansion is possible the binding and removal of each substance from each of the cavity sections or holding sections can be conducted with ease.

For example, even if the positioning of the cavity sections or holding sections on the base members is not carried out at a high density or high level of integration in the first dimension at the first integration, by integrating the base member a second time a high level of compactness or integration is achieved in the second dimension. By separating the integration in this manner, into a two step process, the integration can be carried out simply, rapidly and reliably, and moreover at low cost.

If the positioning of the cavity sections or holding sections on the base member is able to be carried out at a high density or high level of integration in the first dimension at the first integration, then when the base member is further integrated two dimensionally at the second integration, even higher levels of compactness and integration can be achieved.

A seventeenth aspect of the invention is a permeable membrane according to the sixteenth aspect of the invention, wherein the base member is rolled, laminated or arranged in such a way that either enables or prevents expansion, while bringing side portions thereof into contact with each other or while sandwiching an auxiliary member, to give integration and formed into a substantially flat sheet.

The seventeenth aspect of the invention enables the simple formation of a permeable membrane. Furthermore when the base member is expanded, each substance can be bonded or removed from the corresponding cavity section simply, rapidly, and reliably.

An eighteenth aspect of the invention is a permeable membrane according to either one of the sixteenth or seventeenth aspects of the invention, wherein the permeable membrane is provided with a binding section for binding the base member and the auxiliary member in such a way that is either releasable or non-releasable.

As described above the binding section may be either a section which houses and thus binds the base members, or a section which comprises an adhesive portion, as described in the nineteenth aspect of the invention. In those cases where the base members are housed and thus bound, by prescribing a standard size and generating a cartridge, handling is simplified and costs can also be reduced.

With the eighteenth aspect of the invention, by providing the binding section for binding the base member in such a way that is either releasable or non-releasable, the substrate can be integrated to either enable or prevent expansion.

A nineteenth aspect of the invention is a permeable membrane according to the eighteenth aspect of the invention, wherein the binding section is an adhesive portion which bonds side portions of the base member and/or the auxiliary member in such a way that these are either releasable or non-releasable.

With the nineteenth aspect of the invention, by providing an adhesive portion as the binding section, the base member can be bound simply and reliably.

A twentieth aspect of the invention is a permeable membrane according to any one of the sixteenth through nineteenth aspects of the invention, wherein the thread shaped, cord shaped, tape shaped or rod shaped long and slender base member is provided with cavity sections comprising open ended channels and/or bottomless apertures and/or capillaries along the direction of a normal line of the layer surface.

With the twentieth aspect of the invention, by providing channels or apertures across the thickness of the base members, permeable membranes which are easy to handle can be obtained with ease.

A twenty first aspect of the invention is a permeable membrane according to any one of the sixteenth through twentieth aspects of the invention, wherein a linear homoiothermal member is provided inside the base member and/or the auxiliary member for heating or cooling purposes.

The heating or cooling linear homoiothermal member may be electrical heating wires, or a fluid passage through which a heating or cooling medium is passed. Furthermore, the homoiothermal member should preferably be provided along the length of the cavity member or the auxiliary member.

With the twenty first aspect of the invention, by embedding a linear homoiothermal member inside the base member for heating or cooling purposes, a permeable membrane can be provided which is compact and easy to handle, and for which heating or cooling can be carried out efficiently.

A twenty second aspect of the invention is a method of manufacturing an integrated support, comprising a positioning step for positioning and fixing a substance for detection of a predetermined chemical structure at a predetermined location at least one base member, and an integration step for rolling, laminating or arranging said base member to give integration, and the location of respective substances for detection and respective chemical structures are made to correspond.

A twenty third aspect of the invention is a method of manufacturing an integrated support according to the twenty second aspect of the invention, wherein the base member is formed from a thread shaped, string shaped, tape shaped, or rod shaped long and slender member.

A twenty fourth aspect of the invention is a method of manufacturing an integrated support according to either one of the twenty second and twenty third aspects of the invention, wherein in the positioning step, each suspension or semiliquid incorporating a substance for detection with a predetermined chemical structure is positioned by being painted, dispensed, imprinted, suctioned, impregnated or stored onto the base member at a location which corresponds to the chemical structure.

A twenty fifth aspect of the invention is a method of manufacturing an integrated support according to any one of the twenty second through twenty fourth aspects of the invention, wherein in the integration step the base member is rolled, laminated or arranged in such a way that either enables or prevents expansion while bringing the base member into contact with itself or while maintaining a spacing or while sandwiching an auxiliary member, to give integration.

A twenty sixth invention is a method of manufacturing an integrated support according to any one of the twenty second, the twenty fourth, and the twenty fifth aspects of the invention, wherein the base member is formed as a film or thin sheet, the substances for detection are positioned on the base member in approximate lines which do not intersect or contact the other substances, and the integration step involves rolling, laminating or arranging in a way that either enables or prevents expansion to give integration, and wherein a cutting step is provided following the integration step, in which the integrated base member on which the substances for detection are fixed, is sliced thinly to form a plurality of integrated supports in which the cross-sectional surface of the cut functions as a layer surface.

In the above description the expression "approximate lines which do not intersect or contact the other substances" refers to the case, for example, where substantially parallel lines are formed at each predetermined location. These lines need not necessarily be straight, and curved lines could equally well be formed. The term "approximate lines" incorporates long and slender shapes such as thread-like, cord-like and rod-like shapes. It is preferable that during the integration step the base members are rolled, laminated or arranged, without bending the aforementioned lines.

The top and bottom surfaces of the base members are brought in contact with the base member being rolled, laminated or arranged either with a maintained spacing or sandwiching an auxiliary member. The cutting should preferably be performed so as to cut across the lines. For example the cutting can be conducted either perpendicularly to, or at a predetermined angle with respect to the lines.

The twenty third aspect of the invention relates to the integration of a thread shaped or cord shaped base member. In such cases, because the base member is originally thread shaped or cord shaped, then there is no necessity for conducting cutting or processing following the supporting of each substance for detection, and consequently the manufacturing process is simple. Furthermore, because no cutting or processing is necessary there is no danger of losing any of the substances for detection meaning reliability is high.

The twenty sixth aspect of the invention relates to the integration of film-like or thin sheet type base members. In such cases, because the surface area and length onto which each substance for detection is positioned via dispensing is comparatively large, the positioning can be carried out efficiently and easily. Furthermore, in those cases where cavity sections of capillaries, or holding sections of a porous material, a foam material, a fibrous material, or an impregnating material are provided in lines, the suspensions which incorporate the substances for detection can be readily drawn into, impregnated, or stored in the cavity sections or holding sections through capillarity.

Consequently, the integrated supports can be manufactured easily, rapidly, cheaply, and reliably. Moreover, the cutting process enables the simple and rapid manufacture of large quantities of integrated supports, which reduces the cost even further.

A twenty seventh aspect of the invention is a method of manufacturing an integrated support according to any one of the twenty second through twenty sixth aspects of the invention, wherein in the positioning step each suspension or semiliquid incorporating a substance for detection with a predetermined chemical structure, is positioned by being painted, dispensed, imprinted, drawn up, impregnated or stored, onto the base member, or, into a plurality of cavity sections of channels, apertures or capillaries, or into holding sections having a porous material, a foam material, a fibrous material, a material with an irregular surface or an impregnating material, provided on the base member.

In the case where the base member is formed as a film or thin sheet the cavity sections or holding sections are provided in approximate lines. The holding sections are long and slender in form such as thread shaped, cord shaped, tape shaped or rod shaped. It is preferable that pores, foams and so on in the base member between holding sections are removed with the use of an adhesive or the like.

With the twenty seventh invention, in those cases in the positioning step where the cavity sections are formed of capillaries, or in the case where holding sections are formed, the suspensions are positioned by fastening, at the corresponding location, either the capillaries which contain or which have drawn up the required suspensions, or the holding sections where the suspensions have been impregnated and drawn up or stored, and consequently the positioning can be carried out simply and reliably.

With the twenty second, twenty third, twenty fourth, twenty fifth, and twenty seventh aspects of the invention, the substances for detection are positioned and fixed with the base members in an expanded state, with integration then being carried out subsequently. Consequently, by integrating a system in which each substance for detection has been simply, rapidly and reliably positioned and supported on each cavity section or holding section in each location on the base member, large quantities of the integrated members are able to be manufactured simply, rapidly, and moreover at low cost.

Even if, for example, the fixation and positioning of the substances for detection on to the base member is not conducted at a one-dimensionally high level of density or integration, by integrating the base member a two-dimensional high degree of compactness or integration is achievable. By carrying out the integration in this manner, with two separate stages, the integration can be completed simply, quickly, reliably, and moreover at low cost.

If, for example, the fixation or positioning of the substances for detection onto the base member is able to be carried out at a one-dimensionally high level of density or integration, then the subsequent two-dimensional integration of the base member will enable an even higher degree of compactness and integration to be achieved.

A twenty eighth aspect of the invention is a method of manufacturing an integrated support according to either one of the twenty second and twenty seventh aspects of the invention, wherein in the integrating step the base member and/or auxiliary member are bound in such a way that is either releasable or non-releasable.

With the twenty eighth aspect of the invention, the base member is able to be easily integrated with expansion either enabled or prevented.

A twenty ninth aspect of the invention is a method of manufacturing an integrated support according to any one of the twenty second through twenty eighth aspects of the invention, wherein in the positioning step, the substances for detection are fixed and supported onto the base member by drying the positioned suspensions or semiliquids which contain the substances for detection.

With the twenty ninth aspect of the invention, by using a dispensing device, the substances for detection positioned on the base member can be quickly and simply fixed and bonded.

A thirtieth aspect of the invention is a method of manufacturing a DNA integrated support comprising: a positioning step in which at each of a plurality of predetermined locations positioned along the length of at least one thread shaped, cord shaped, tape shaped or rod shaped long and slender base member, a suspension or semiliquid incorporating a genetic substance such as an oligonucleotide with a predetermined base sequence which corresponds with that particular location is positioned and fixed onto the base member by being painted, dispensed, imprinted, drawn up, impregnated or stored, either into cavity sections provided on the base member comprising channels, apertures or capillaries, or into holding sections provided on the base member having a porous material, a foam material, a fibrous material, a material with an irregular surface, or an impregnating material; and an integration step wherein the base member, on which is positioned the suspensions or semiliquids, is rolled, laminated or arranged in such a way that either enables or prevents expansion, while bringing side portions of the base member into contact with each other or while maintaining a spacing or while sandwiching an auxiliary member, to give integration.

A thirty first aspect of the invention is a method of manufacturing a DNA integrated support comprising: a positioning step in which at each of a plurality of predetermined locations side by side on at least one film type or thin sheet type base member, a suspension or semiliquid incorporating a genetic substance such as an oligonucleotide with a predetermined base sequence which corresponds with that particular location is positioned and fixed onto the base member in substantially parallel lines, into cavity sections comprising channels, apertures or capillaries and which are provided in substantially parallel lines on the base member, or into thread shaped, cord shaped, tape shaped or rod shaped long and slender holding sections which are provided in substantially parallel lines on the base member and which have a porous material, a foam material, a fibrous material, a material with an irregular surface, or an impregnating material by being painted, dispensed, imprinted, drawn up, impregnated or stored;
an integration step wherein the base member, on which is positioned the suspensions or semiliquids, is rolled, laminated or arranged in such a way that either enables or prevents expansion and so as not to bend the substantially parallel lines, while bringing face pairs of the base member into contact with each other or while maintaining a spacing or while sandwiching an auxiliary member, to give integration; and a cutting step wherein the integrated base member is cut thinly across the substantially parallel lines to obtain a plurality of DNA integrated supports in which the cross-sectional surface of the cut functions as a layer surface.

A thirty second aspect of the invention is a method of manufacturing an integrated minute vessel comprising: a processing step for providing on at least one base member, either a plurality of cavity sections such as channels with bottoms or ends, or holding sections having a porous material, a foam material, a fibrous material, a material with an irregular surface, or an impregnating material; and an integration step wherein the base member is rolled, laminated or arranged in such a way that either enables or prevents expansion, while bringing the base member into contact with itself or while maintaining a spacing or while sandwiching an auxiliary member, to give integration.

In the description, the formation of the "cavity sections such as channels" can be conducted either by removing material from the base member, or by adding additional material to the base member.

A thirty third aspect of the invention is a method of manufacturing an integrated minute vessel according to the thirty second aspect of the invention, wherein the base member is formed as a film or thin sheet, and the cavity sections or the holding sections are each provided in approximate lines which do not intersect or contact the other sections, and wherein a cutting step for cutting the integrated base member to form a plurality of integrated minute vessels is provided after the integration step.

In the integration step it is preferable that the rolling, laminating or arranging is conducted without bending the approximate lines. It is also possible to form cavity sections of open-ended channels or bottomless apertures, and then subsequently close one end of each cavity section. The cutting should preferably be conducted to cut across the lines.

A binding step may also be provided following the integration step, wherein the base member and auxiliary member are bound in such a way that is either releasable or non-releasable.

With the thirty second and thirty third aspects of the invention, following the formation of the plurality of cavity sections such as channels on the base member, the base member is rolled, laminated or arranged to either enable or prevent expansion, to give integration. Consequently, integrated minute vessels can be formed simply, rapidly, at low cost, and moreover in large volume.

A thirty fourth aspect of the invention is a method of manufacturing a permeable membrane comprising: a processing step for providing on at least one base member, either a plurality of cavity sections such as bottomless or open-ended channels, or holding sections having a porous material, a foam material, a fibrous material, a material with an irregular surface, or an impregnating material; and an integration step wherein the base member is rolled. laminated or arranged in such a way that either enables or prevents expansion, while bringing the base member into contact with itself or while maintaining a spacing or while sandwiching an auxiliary member, to give integration.

In the description, the formation of the "cavity sections such as channels" can be conducted either by removing material from the base member, or by adding additional material to the base member.

A thirty fifth aspect of the invention is a method of manufacturing a permeable membrane according to the thirty fourth aspect of the invention, wherein the base member is formed as film or thin sheet, and the cavity sections or the holding sections are each formed in approximate lines, and wherein a cutting step for cutting the integrated base member to form a plurality of permeable membranes is provided after the integration step.

In such a case, in the integration step it is preferable that the rolling, lamination or arrangement is conducted without bending the approximate lines of the cavity sections or holding sections. The cutting step should preferably be conducted so as to cut across the lines, so that, for example, the cutting occurs either perpendicularly to, or at a predetermined angle with respect to the lines. A binding step can also be provided following the integration step, wherein the base member and auxiliary member are bound in such a way that is either releasable or non-releasable.

With the thirty fourth and thirty fifth aspects of the invention, following the formation of the plurality of cavity sections such as channels on the base member, the base member is rolled, laminated or arranged to either enable or prevent expansion, to give integration. Consequently, permeable membranes can be formed with ease.

A thirty sixth aspect of the invention is a method of using an integrated medium, wherein by passing a heating fluid or a cooling fluid through an integrated support, an integrated minute vessel, or a permeable membrane according to any one of the first through twenty first aspects of the invention, the integrated support, integrated minute vessel, or permeable membrane is heated or cooled respectively.

With the thirty sixth aspect of the invention the integrated medium can be heated or cooled simply and rapidly.

A thirty seventh aspect of the invention is a method of using an integrated medium, comprising: a processing step for conducting processing using an integrated support, an integrated minute vessel, or a permeable membrane according to any one of the first through twenty first aspects of the invention, and a measurement step for conducting measurements of an optical state with the processed integrated support, integrated minute vessel, or permeable membrane, either in an expanded state or in an integrated state.

With the thirty seventh aspect of the invention, measurements can be conducted in either the expanded state or the integrated state depending on the conditions, thus enabling diverse use.

A thirty eighth aspect of the invention is a method of using an integrated medium according to the thirty seventh aspect of the invention, wherein the measurement in the measurement step with the integrated support, integrated minute vessel, or permeable membrane in an integrated state involves identification of an absolute location on the layer surface thereof.

With the thirty eighth aspect of the invention, because the absolute location on the substrate can be identified, the measurements can be conducted efficiently and rapidly on the integrated state.

A thirty ninth aspect of the invention is a method of using an integrated minute vessel comprising: a hardening step wherein with a base member of the integrated support, integrated minute vessel, or permeable membrane according to any one of the first through twenty first aspects of the invention in an expanded state, a predetermined suspension is positioned in a predetermined cavity section or holding section by dispensing, imprinting, impregnating, painting, or storing, and subsequently hardened; an integration step for integrating the base member on which the predetermined suspensions have been hardened, so that expansion is either enabled or prevented; a fluidization step for fluidizing the hardened suspensions inside the respective cavity sections or holding sections; and a processing step for conducting reaction processing within the cavity sections or holding sections.

With the thirty ninth aspect of the invention, because integration of the base member is conducted following hardening of the suspensions, the integration is completed easily. Furthermore, because the reaction processing is carried out following fluidization of the suspensions, the reaction processing can be conducted with good reliability. Overall, the processing can be carried out rapidly and reliably.

A fortieth aspect of the invention is a method of using an integrated minute vessel according to the thirty ninth aspect of the invention, wherein a suction step in which a pin shaped liquid passage is inserted into each of the cavity sections or holding sections for drawing up reaction products, is provided after the processing step. It is preferable that the pin shaped liquid passage is capable of both suction and discharge.

With the fortieth aspect of the invention the reaction products can be obtained reliably and simply.

A forty first aspect of the invention is a method of using an integrated minute vessel according to either one of the thirty ninth and fortieth aspects of the invention, wherein the suspensions incorporate magnetic particles, and the suction step is conducted with a magnetic field either applied to, or absent from each of the cavity sections or holding sections. Furthermore, the magnetic particles can also be captured by using a pin with a magnetic tip, and inserting the pin into the cavity sections or holding sections.

With the forty first aspect of the invention, by using magnetic particles combined with the various magnetic particle processing devices and methods, automation can be achieved which is efficient and moreover very reliable.

A forty second aspect of the invention is an integrated medium storing fluid passage, comprising at least one fluid passage, and a pressure control device for controlling the pressure inside the fluid passage, wherein an integrated support, an integrated minute vessel or a permeable membrane according to any one of the first through twenty first aspects of the invention is stored either inside the fluid passage, or inside a storage section communicated with the fluid passage, and the fluid is able to pass through the integrated support, integrated minute vessel or permeable membrane, or the fluid is able to contact the integrated support, integrated minute vessel or permeable membrane.

Methods for storing the integrated support or the like inside the fluid passage include the case where the direction of the normal line of the substrate and the direction of the passage of the fluid are approximately the same, as well as the case where the direction of the normal line of the substrate and the direction of passage of the fluid are approximately orthogonal.

With the forty second aspect of the invention, highly efficient reaction processing can be conducted with the substances supported and stored on the integrated medium.

A forty third aspect of the invention is an integrated medium storing fluid passage according to the forty second aspect of the invention, wherein the fluid passage, or the fluid passage and storage section are detachable with respect to the pressure control device, or the storage section or the integrated support, integrated minute vessel, or permeable membrane are detachable with respect to the fluid passage or storage section, and wherein the fluid passage and the pressure control device comprise a displacement device for conducting relative displacement between vessels of the fluid passage, as well as a dispensing device.

With the forty third aspect of the invention, by providing the fluid passage so as to be detachable with respect to the pressure control device, automatic processing of the dispensing device becomes a possibility, and moreover handling is simplified.

A forty fourth aspect of the invention is an integrated medium storing fluid passage according to the forty second aspect of the invention, wherein the pressure control device comprises a nozzle for controlling pressure by drawing gas from or discharging gas into the fluid passage, and the fluid passage comprises a reservoir section which stores fluid and which is joined in a detachable manner to the nozzle, and a narrow diameter section which is communicated with the reservoir section, and which is of smaller diameter than the reservoir section and is able to be inserted inside a vessel.

With the forty fourth aspect of the invention, by making the fluid passage detachable with respect to the nozzle which functions as the pressure control device, the entire fluid passage, including the integrated medium contained therein, can be handled, making handling even easier.

A forty fifth aspect of the invention is an integrated medium storing fluid passage according to any one of the forty second through forty fourth aspects of the invention, wherein a light emitting device for irradiating light on to the integrated support, integrated minute vessel, or permeable membrane stored in the fluid passage or the storage section, and/or a light reception device for capturing light emitted from the integrated support, integrated minute vessel, or permeable membrane are provided. The light emitting device is necessary in those cases where, for example, the marking substances are fluorescent substances and need to be irradiated with an excitation beam.

With the forty fifth aspect of the invention, measurements on the integrated medium are able to be conducted simply and accurately.

A forty sixth aspect of the invention is an integrated medium storing fluid passage according to the forty fifth aspect of the invention, further comprising a rotational drive section for rotational driving the fluid passage and the storage section, or the integrated support, integrated minute vessel, or permeable membrane contained therein.

This aspect of the invention is necessary when light irradiation and light capture is conducted for only one region of the integrated medium.

With the forty sixth aspect of the invention, measurement of the entire integrated medium is possible even in those cases where light irradiation and light capture is conducted for only one region of the integrated medium.

A forty seventh aspect of the invention is an integrated medium storing fluid passage according to any one of the forty second through forty sixth aspects of the invention, comprising a cooling medium or a heating medium provided outside of the fluid passage so as to be movable towards or away from an external wall of a neighboring fluid passage which contains the integrated medium, in order to heat or cool the integrated support, integrated minute vessel, or permeable membrane stored inside the fluid passage or inside the storage section communicated with the fluid passage.

With the forty seventh aspect of the invention, heating or cooling of the integrated support, integrated minute vessel, or permeable membrane stored inside the fluid passage of any of the aspects of the invention can be carried out easily, and with a simple construction.

A forty eighth aspect of the invention is an integrated medium storing fluid passage according to any one of the forty second through forty seventh aspects of the invention, further comprising a magnetic device which enables the application of or removal of a magnetic field acting in the direction of a normal line of the substrate, onto the inside of each of the cavity sections of the integrated support, integrated minute vessel, or permeable membrane stored inside the fluid passage or the storage section.

With the forty eighth aspect of the invention, a magnetic field can be easily and reliably applied to, or removed from each of the cavity sections of the integrated support, integrated minute vessel, or permeable membrane stored inside the aforementioned fluid passage.

A forty ninth aspect of the invention is a magnetic separation device which comprises a mounting section on which is mounted an integrated support, integrated minute vessel, or permeable membrane according to any one of the first through twenty first aspects of the invention, and a magnetic section which is provided on the lower surface of the mounting section, and which enables either the application of, or removal of a magnetic field, from beneath the substrate, with respect to the inside of each of the cavity sections of the integrated support, integrated minute vessel, or permeable membrane.

With the forty ninth aspect of the invention, a magnetic field can be easily and reliably applied to, or removed from each of the cavity sections of the integrated support, integrated minute vessel, or permeable membrane.

### BRIEF DESCRIPTION OF THE DRAWINGS

FIG. 1 is a plan view showing a disk shaped integrated support or the like according to a first embodiment of the present invention.
FIG. 2 is a partial enlargement of the disk shaped integrated support or the like according to the first embodiment of the present invention.
FIG. 3 is a partial enlargement of a DNA integrated support according to a first embodiment of the present invention.
FIG. 4 is a diagram showing a dispensing device according to a second embodiment of the present invention.
FIG. 5 is a diagram showing a dispensing device according to a third embodiment of the present invention.
FIG. 6 is a descriptive diagram showing the use of the dispensing device according to the third embodiment of the present invention.
FIG. 7 is a diagram showing a dispensing device according to a fourth embodiment of the present invention.
FIG. 8 is a diagram showing an integrated minute vessel according to a fifth embodiment of the present invention.
FIG. 9 is a diagram showing an integrated support according to a sixth embodiment of the present invention.

### DETAILED DESCRIPTION OF THE PREFERRED EMBODIMENTS

As follows is a description of embodiments of the present invention, based on the drawings. It should be noted that the embodiments described here do not limit the invention in any way unless particularly specified.

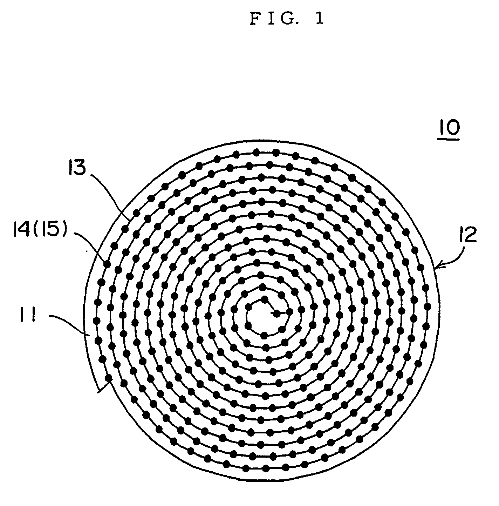

FIG. 1 is a diagram showing an example of an integrated support (or an integrated minute vessel or a permeable membrane, hereafter referred to as an "integrated medium") 10 according to a first embodiment, viewed from the layer surface (the integrated surface) thereof.

The integrated medium 10 comprises a substantially flat circular substrate 12 which is formed by bringing into contact the side portions (non-integrated surface) 13 of a single flexible cord shaped long and slender base member 11, by rolling and integrating the base member 11 so that expansion is either enabled or prevented. Suitable materials for the base member 11 include organic materials such as resins such as polyethylene, polystyrene, polypropylene, and urethane, inorganic materials such as semiconductors, metals, metalloids, glass fiber, and ceramics, as well as organic/inorganic mixed materials such as fine grains or ultra fine grains of a metal or a ceramic laid on the surface of a tape type or film type organic material.

In the case where the integrated medium 10 is used as a DNA integrated support, the diameter of the substrate 12 is of the order of several mm to several cm, and the thickness between approximately 0.1mm and several mm. A plurality of cavity sections 14 (channels 15) which either have bottoms or are bottomless are provided at a predetermined spacing along the length of the side portions (non-integrated surface) of the base member 11.

The size or spacing occupied by a substance for detection at each fixed location can be within the range of 0.1µm to several mm. Even if, for example, the spacing between fixed locations on the base member 11 is large and the one dimensional density is low prior to integration, the two dimensional surface following integration increases the density of the fixed locations.

The number of cavity sections 14 can be set as required within a range from several thousand to more than several hundred thousand, for example. The substrate 12 is integrated by rolling, laminating or arranging the base member 11 to give integration. A plurality of cavity sections 14 (apertures) which either have bottoms or are bottomless are formed between the side portions of the base member 11, and the mouths of the cavity sections are arranged on a spiral along the length of the base member on the surface of the substrate 12, that is, on the layer surface.

An adhesive portion such as a velcro surface on which is formed a plurality of minute irregularities is provided on the side portions of the base member 11 as a binding section which will bond in a releasable manner. In such a case, it is also possible to form no cavity sections and to use the minute irregularities as holding sections to fix the substances for detection on the side portions of the base member 11.

In such a case, even if, for example, the side portions of the base member 11 are brought in contact by rolling, laminating or arranging to give integration, because a plurality of minute irregularities exist between adjacent sections of the base member 11, the substances for detection can be exposed on the layer surface even if a spacing is not maintained between the sections of the base member 11. Furthermore, even though the side portions of the base member 11 may appear as smooth as a glass surface, a multitude of microscopic irregularities can be used as holding sections, enabling the exposure of the substances for detection fixed on the side portions.

In the case where numeral 10 represents a DNA integrated support, an oligonucleotide with a base sequence which corresponds with a substance for detection (a probe) is fixed and supported in each cavity section 14. The base sequence corresponds with the aforementioned chemical structure. The location of each cavity section 14 corresponds with the base sequence.

In such cases, each oligonucleotide is not merely fixed to the surface of the substrate 12, but is rather supported in a cavity section 14 at the location which corresponds with the base sequence of that particular oligonucleotide. Consequently, because the bonding surface area can be increased, and the amount of adhesion thus increased, the reaction efficiency can be improved and the reliability of the bonding improved, rather than simply supporting the oligonucleotides in identical arrangements on a flat substrate.

Furthermore, in those cases where the side portions of the base member 11 are bonded in a manner which is releasable, the state of the substrate 12 and the cavity sections 14 can be altered between an expanded (unfolded) state and an integrated state. By expanding the integrated medium 10 into the base member 11, the surface area of each cavity section 14 increases, and the spacing between cavity sections 14 also increases in comparison with the integrated state, making the work of positioning or bonding each substance for detection using dispensing techniques relatively simple.

In contrast, for example in the case of the determination of an unknown base sequence for a genetic substance, a reaction process is carried out by contacting a marked genetic substance as the target substance with a suspended liquid. In such a case, if the reaction is conducted with the integrated medium 10 in an integrated state, then because the migration of, and contact of the suspension with respect to the integrated medium 10 can be carried out efficiently and rapidly within a small region, the reaction efficiency can be improved.

Furthermore in the case of measurement analysis, measurement analyses can be conducted in both the expanded and the integrated states. When measurements are conducted on the integrated state, then measurements of the entire integrated medium 10 can be made with ease. Similarly, measurements conducted on the expanded state enable a reliable and accurate grasp to be made of the conditions within each cavity section of the integrated medium 10.

In the case where numeral 10 represents an integrated minute vessel, each cavity section 14 is formed-with a bottom. In such cases, when small quantities of a liquid are being dealt with, because an integrated system can be used in which the minute vessels match the volume of the liquids, handling is possible without increasing the bulk unnecessarily, thus contributing to space saving.

Furthermore even in such cases, by altering the state of the substrate 12 as required, through each of the cavity sections 14, either to the expanded state or the integrated state, a variety of gains can be achieved. For example, in the case where a variety of substances are to be stored in each of the cavity sections 14, then the operation is more easily completed in the expanded state. Depending on the particular material to be stored, if the material is able to be gelatinized (hardened) and solated (fluidized), then by carrying out the storage with the material in a solid or semiliquid state and then fluidizing the material following integration, handling is simplified.

Furthermore, if reaction processing is then conducted using the integrated state, and the removal of each substance conducted in the expanded state, then handling is simple, reliable, rapid, and efficient. Measurement analyses can be conducted on both the integrated state and the expanded state.

Furthermore, it is also possible to increase the contact efficiency of a fluid with the cavity sections 14, if in addition to providing the cavity sections 14 with bottoms between the base members 11, bottomless apertures are provided for each base member 11 through which fluids are able to pass.

In the case where numeral 10 represents a permeable membrane, each cavity section 14 is bottomless. In such cases, the substrate 12 can still be altered between the integrated state and the expanded state through each cavity section 14, as is required. For example, in the case where a variety of substances are to be bonded in each of the cavity sections 14 in order to manufacture a variety of filters with various properties, then conducting the bonding in the expanded state is both simpler and more reliable.

Furthermore, when allowing a liquid to permeate through the substrate, carrying out the operation with the substrate in the integrated state enables the reaction process to be more rapid, more efficient, simpler, and more accurate. The removal of each substance is most efficiently conducted in the expanded state.

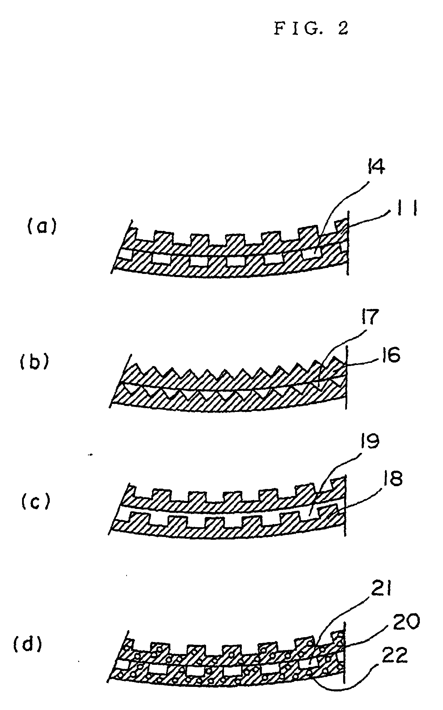

FIG. 2 (a) shows an enlarged model of the base member 11 of the integrated medium 10 shown in FIG. 1, and cavity sections 14 with square shaped openings which are formed on the side portions of the base member 11. In the case shown, an attachment section can also be provided as a binding section for bonding the side portions of the base member 11 together in a way that is either releasable or non-releasable.

FIG. 2 (b) shows a base member 16 of another integrated medium, and cavity sections 17 with triangular shaped openings which are formed on the side portions of the base member 16. In comparison with FIG. 2 (a), the case shown is able to accommodate a greater density of cavity sections 17. In the case shown, the side portions of the base member 16 are brought in contact when the base member is rolled, laminated or arranged, but then rather than being bound by an adhesive portion, are bound in a way that is either releasable or non-releasable by storage inside a storage section.

FIG. 2 (c) shows a base member 18 of another integrated medium, and channels (cavity sections) 19 which are formed on the side portions of the base member 18. In the example shown, the base member 18 is integrated and formed into a substantially flat sheet by rolling with a spacing maintained between the side portions of the base member 18.

Consequently in the example shown, each channel (cavity section) 19 becomes an integral portion of the spacing between the side portions. In the example shown, either the base member 18 needs to be formed of an inflexible material, or the integrated medium needs to be stored in a storage section.

In the example shown, because liquids are able to pass through the aforementioned spacing, the encounter rate can be increased within each cavity section 19 between the substances contained in the liquids and the substances bonded to each of the cavity sections 19, and the contact efficiency improved.

FIG. 2 (d) shows a base member 20 of another integrated medium, and cavity sections 21 which are formed on the side portions of the base member 20. In the example shown, channels (cavity sections) 21 are formed at intervals on the base member 20, and the base member 20 is formed of a porous material in which a multitude of apertures penetrate through the material, meaning the base member 20 itself is also provided with apertures 22 (cavity sections). The apertures (cavity sections) 22 are used for enabling liquids to permeate from top to bottom.

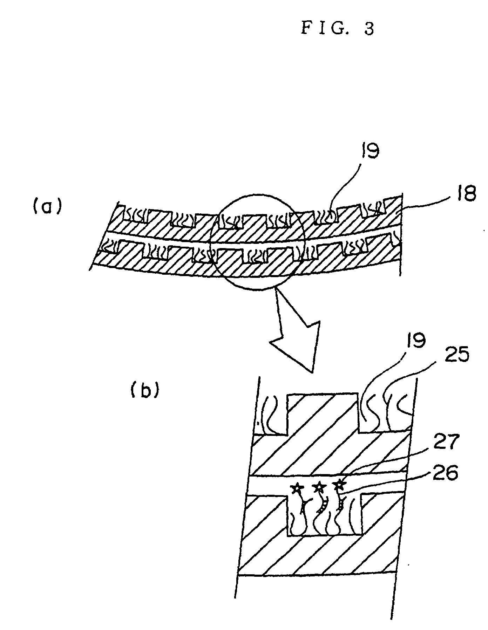

FIG. 3 is an integrated medium wherein for each of the cavity sections 19 provided on the side portions of the base member 18 an oligonucleotide 25 with a base sequence which corresponds to the location of that cavity section 19 is bonded to the side surface of the cavity section 19 as a substance for detection (probe). FIG. 3 (b) shows a model of a particular cavity section 19 to which has been bonded an oligonucleotide 25, which has been hybridized with a single strand DNA fragment target material of a predetermined base sequence, the DNA fragment having been marked with a light emitting material 27 such as a fluorescent substance.

In the example shown, a spacing exists between the base member 18 of the integrated support, and so by passing a liquid through the spacing, contact is promoted between the suspended liquid of the aforementioned target substance and the aforementioned substance for detection.

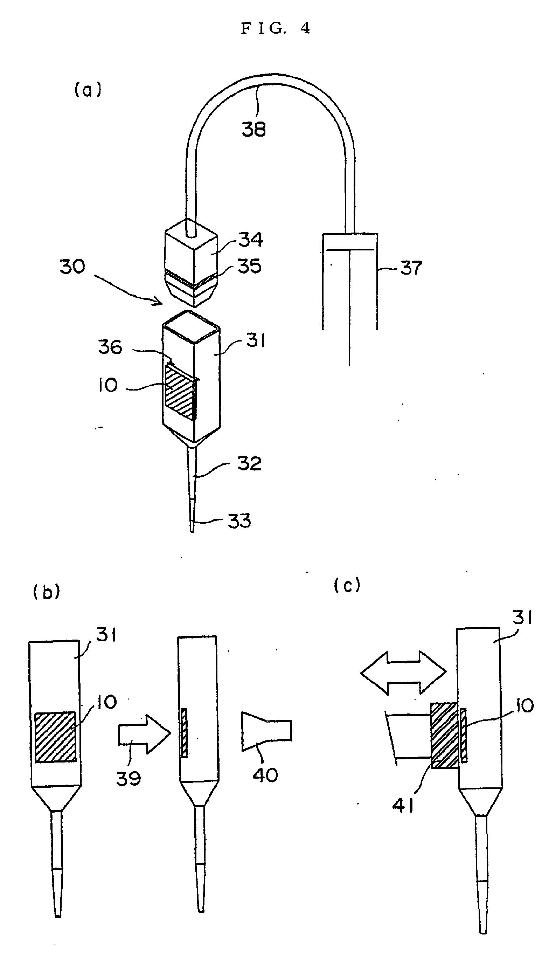

FIG. 4 shows a dispensing device 30 according to a second embodiment, which is an example of the fluid passage used for bringing the integrated medium 10 into contact with a suspension.

As shown in FIG. 4 (a), the dispensing device 30 houses the integrated medium 10, and comprises: a hollow, approximately square cylinder shaped connected storage section 31 which is connected in a detachable manner to a suction discharge mechanism such as a cylinder for conducting the suction and discharge of liquids, a narrow diameter section 32 which is of a smaller diameter than the connected storage section 31 and which is moreover able to be inserted inside a vessel, and a tapered tip section 33.

In addition to use with the integrated medium 10 described above, this particular embodiment can also be used by attaching conventional DNA chips. By forming the connected storage section 31, the narrow diameter section 32 and the tip section 33 as a tip portion which functions as the aforementioned fluid passage, and housing the integrated medium 10 inside the tip portion and creating a cartridge, handling can be simplified considerably.

The connected storage section 31 is formed so as to enable mounting on a nozzle 34 which carries out the suction and discharge of liquids. The nozzle 34 is provided with an O-ring 35 to prevent liquid leakage, and is connected by a flexible pipe 38 to a cylinder 37 which functions as the suction discharge mechanism. The integrated medium 10 is attached to the connected storage section 31 by attaching the layer surface (substrate surface) of the integrated medium 10 to an attachment section 36 on the wall of the connected storage section 31.

FIG. 4 (b) shows an example case where measurements are to be conducted on the integrated medium 10. For the case shown, in those cases where the marking substance is a fluorescent material, a light emission section 39 for irradiating an excitation beam of light, and a light reception section 40 for capturing fluorescent light from the connected storage section 31 are provided facing the wall surfaces of the connected storage section 31 to which is attached the layer surface of the integrated medium 10.

In those cases where the marking substance is a chemiluminescent material, the light emission section is unnecessary. Consequently, the reaction processing of the integrated medium 10, and the marking substance distribution produced as a result of the reaction processing is able to be measured.

FIG. 4(c) shows an example of heating or cooling of the integrated medium 10. In the example shown, a heating or cooling homoiothermal device 41 is provided facing the wall surface of the connected storage section 31 to which is attached the layer surface of the integrated medium 10, and is able to be moved closer to, or further away from the wall surface. By so doing, the reaction processing of the integrated medium 10 can be accelerated simply and efficiently .

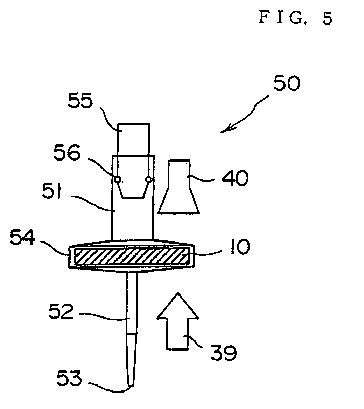

FIG. 5 shows another dispensing device 50 according to a third embodiment.

The dispensing device 50 comprises: a hollow, approximately cylindrical reservoir section 51 which stores the liquid that has been drawn up, and which is attached to a suction discharge mechanism such as a cylinder for carrying out suction and discharge of liquids; a storage section 54 which is communicated with the reservoir section 51 and which stores the disk shaped integrated medium 10; a narrow diameter section 52 which is communicated with the storage section 54 and is of a smaller diameter than the reservoir section 51, and which is moreover able to be inserted inside a vessel; and a tapered tip section 53.

The reservoir section 51 is mounted detachably to a nozzle 55 which is communicated with the cylinder of the suction discharge mechanism and carries out the suction and discharge of liquids. The nozzle 55 is provided with an O-ring 56 around the perimeter of the nozzle 55 to prevent liquid leakage. By forming the reservoir section 51, the storage section 54, the narrow diameter section 52, and the tip section 53 as a tip portion which functions as a fluid passage through which fluids can flow, and then housing the integrated medium 10 inside the tip portion and creating a cartridge, handling can be simplified considerably.

Moreover, with this particular embodiment, in the case where a fluorescent material is used as the marking substance for example, a light emission section 39 for irradiating light on a predetermined region of the storage section 54 is positioned below (or above) the storage section 54, while a light reception section 40 is provided above (or below) the storage section 54.

The substrate of the integrated medium 10 is formed in a disk shape, and in the central region, in this particular case in the central region of the disk shape integrated medium 10, is provided a rolling core which functions as an auxiliary member and which has a diameter of approximately the same size as the external diameter of the reservoir section 51, and the base member is rolled about this rolling core.

Furthermore, a rotational apparatus (not shown in the drawings) is also provided to enable the rotational driving of the entire tip section incorporating the storage section 54, or just the storage section 54, or the integrated medium 10 stored inside the storage section 54. Consequently, for all regions of the integrated medium 10, by using hybridization, the optical state of double strands and the like formed on the surface of the substrate are able to be measured.

The light emission section 39 is a light source with a predetermined wavelength which corresponds to the marking substance being used, and in the case where the marking substance is a fluorescent material, is the excitation wavelength for that fluorescent material. The light reception section 40 can be an image pickup device such as a CCD camera, or a high performance fluorescent scanner with a resolution of 1∼10µm for example. It is preferable that such a scanner has an auto focus function.

The storage section 54 can also be formed of a transparent medium, with the transparent medium having a convergent lens effect. Moreover, when conducting measurements, in order to prevent scattering and dissipation of the light due to water droplets generated inside the storage section 54, measurements should be conducted with the storage section filled with purified water.

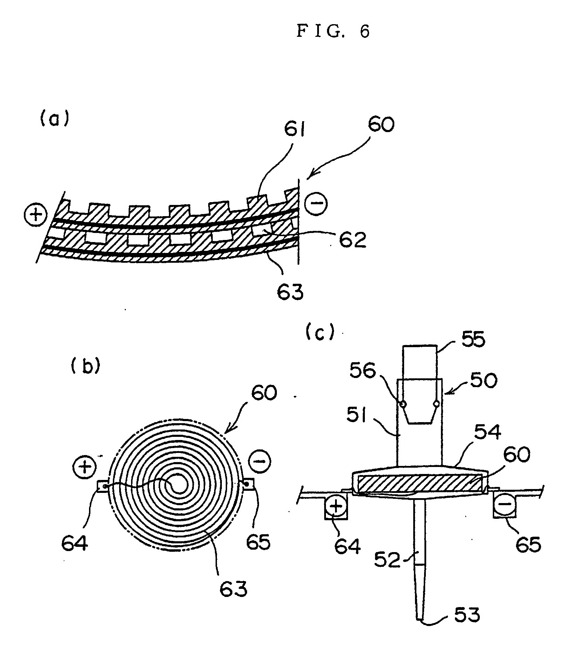

FIG. 6 shows an example of the heating or cooling of the dispensing device 50. FIG. 6 (a) shows a heated integrated medium 60. The heated integrated medium 60 is a substantially flat sheet formed rolling in a manner which is either releasable or non-releasable to bring the side portions of either one, or two or more cord shaped base members 62 into contact.

Furthermore, channels are formed on the base members 62 at a uniform pitch, and by rolling the base members 62 a plurality of cavity sections 62, which either have bottoms or are bottomless, are arranged on a spiral shape on the surface of the substrate. Furthermore, heating wire 63 is embedded inside the base members 61 along the length of the members.

FIG. 6 (b) shows the shape of the heating wire 63, which is connected electrically to terminals 64, 65 which are in turn connected to an electrical power source. FIG. 6 (c) shows a dispensing device 50 with the heated integrated medium 60 stored therein.

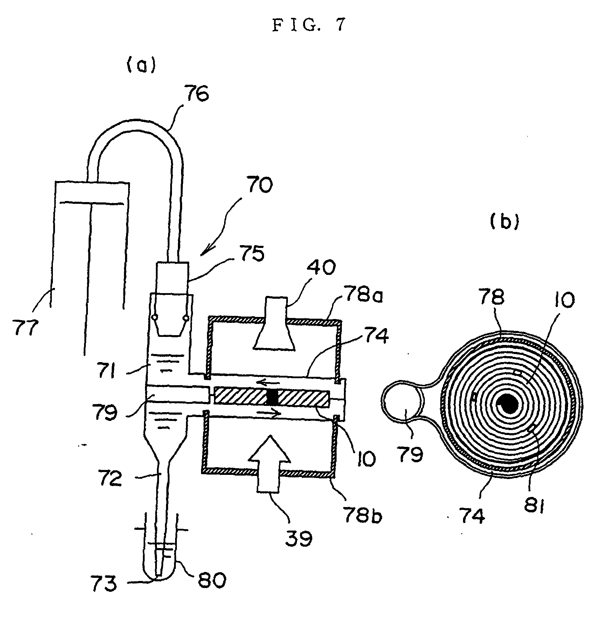

FIG. 7 shows an example of another dispensing device 70 according to a fourth embodiment of the invention.

As shown in FIG. 7 (a), the dispensing device 70 of this particular embodiment comprises a reservoir section 71 which stores a liquid which has been drawn up, and which is mounted in a detachable manner to a nozzle 75 used for carrying out the suction and discharge of liquids, a narrow diameter section 72 which is of a smaller diameter than the reservoir section 71 and which is able to be inserted inside a vessel 80, and which is moreover communicated with the reservoir section 71, and a tapered tip section 73. The nozzle 75 is connected to a cylinder 77 used for carrying out the suction and discharge via a flexible conduit 76.

Moreover the reservoir section 71 is also communicated with a storage section 74 which is provided at the side of the reservoir section 71 and which contains a transparent integrated medium 10. The top surface of the storage section 74 is connected to and covered with a shielding wall 78a which is able to be moved in a vertical direction, while the bottom surface is connected to and covered with a shielding wall 78b which is also able to be moved in a vertical direction.

A light emission section 39 which is able to irradiate the entire storage section 74 from below is provided within the shielding wall 78b, while a light reception section 40 which captures light from above from the entire storage section 74 is provided within the shielding wall 78a.

FIG. 7 (b) is a diagram showing the dispensing device 70 viewed from above to show the integrated medium 10 inside the shielding walls 78a, 78b. A partitioning plate 79 is provided inside the reservoir section 71 so that liquid can not flow directly between the narrow diameter section 72 and the nozzle 75, but will instead be directed through the storage section 74, and permeate the integrated medium 10 to thus enable the passage of liquid between the narrow diameter section 72 and the nozzle 75. Numeral 81 denotes the light emitting medium used as marking on the integrated medium 10.

With this particular embodiment the light emission section 39 is able to irradiate an excitation beam onto the entire integrated medium 10 in a single step, and the light reception section 40 is similarly able to capture light from the entire integrated medium 10, and so measurement of the optical state can be conducted with ease.

Furthermore with this embodiment, in those cases where heating or cooling processing is to be carried out, by replacing the shielding walls 78a, 78b with heating or cooling media which are positioned either proximate to, or touching the storage section 74 from above and below, heating or cooling can be carried out very efficiently.

By housing the integrated medium 10 inside the dispensing device 70 in the manner described above for this embodiment, a variety of operations such as reaction, marking, washing, measurements, and heat processing can be conducted.

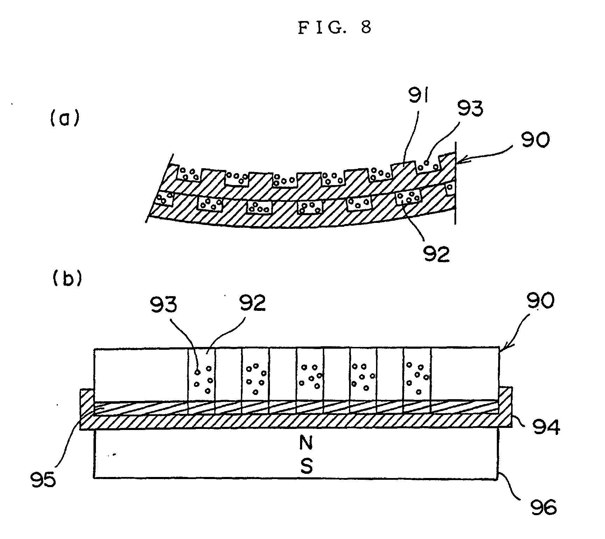

FIG. 8 is a diagram showing an integrated minute vessel 90 which contains magnetic particles, according to a fifth embodiment of the present invention.

FIG. 8 (a) is an enlargement of one section of the integrated minute vessel 90 with magnetic particles 93 stored inside the integrated minute vessel 90, where numeral 91 denotes a base member and numeral 92 a cavity section.

As is shown in FIG. 8 (b) which is a side section schematic view of FIG. 8 (a), each cavity section 92 provided in the integrated minute vessel 90 is sealed by a bottom section 95 with each cavity section 92 thereby forming a minute well. Numeral 94 denotes a mounting section for mounting the integrated minute vessel 90, and beneath the mounting section 94 is provided a magnetic device comprising either a permanent magnet or an electromagnet.

Consequently, in those cases where a magnetic field is to be applied to the magnetic particles 93 stored in each of the cavity sections 92 of the integrated minute vessel 90, the integrated minute vessel 90 is mounted onto the mounting section 94. Furthermore, it is also possible to insert a pin with a magnetized tip into the cavity sections 92 of the integrated minute vessel 90 to capture the magnetic particles. It is also possible to insert a pin like liquid passage (conduit) into the cavity sections 92 to draw up or discharge liquid inside the cavity sections 92.

Next is a description of a method for determining a DNA base sequence using the integrated minute vessel 90 with magnetic particles according to the fifth embodiment of the invention.

A flat surface base member 91 is prepared in which channels (cavity sections) 92 have been formed at a predetermined spacing. Oligonucleotides of predetermined base sequences are fixed to the surface of pre-prepared magnetic particles. Necessary number of magnetic particles are prepared, to the surface of which Oligonucleotides of predetermined base sequences are fixed and which are marked by necessary materials required to represent these base sequences, for example, combining a variety of fluorescent materials with various light emission wavelengths.

A fragment of a single strand, marked DNA with a targeted unknown base sequence is then prepared and suspended in a liquid with the aforementioned magnetic particles. Furthermore, melted agar is then added to the suspension, and the suspension positioned by dispensing into the channels provided on the base member 91, taking care to prevent the agar from solidifying.

Following positioning, the agar containing suspension is gelatinized by cooling to fix the suspension inside the channel. The fixed base member 91 is then integrated by rolling to bring the surfaces together. Following integration, the base member 91 is cut into slices of a predetermined thickness, thus manufacturing a plurality of integrated minute vessels 90 with bottoms, which contain the aforementioned gelatinized suspension.

Next, for each of the integrated minute vessels with bottoms 90, by using the various methods described above to heat the suspension which incorporates gelatinized magnetic particles 93, the suspension is solated and fluidized, thus accelerating the reaction processes. As a result, the oligonucleotide of corresponding base sequence will hybridize to the target single strand DNA fragment.

As a result of the reaction, by irradiating the integrated minute vessels 90 with an excitation beam of light, the fluorescent marking substances will emit light, and the wavelength combination of the emitted light is used to analyze the base sequence.

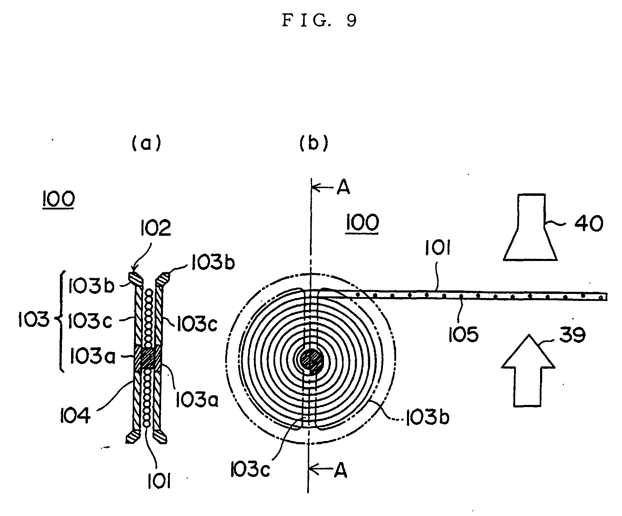

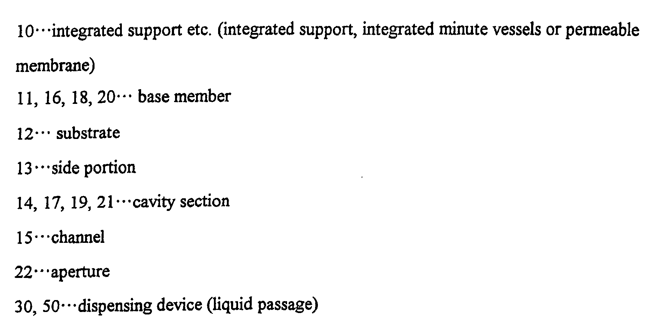

As follows is a description of a method of manufacturing and using an integrated support 100 according to a sixth embodiment of the invention, based on the diagrams of FIG. 9.

FIG. 9 shows the integrated support (DNA integrated support) 100 according to the sixth embodiment of the invention. FIG. 9 (a) is a cross section of the integrated support 100 shown in FIG. 9(b), on the line AA. The integrated support 100 comprises a flexible cord shaped or tape shaped long and slender base member 101, and a rolling reel 102 about which is rolled the base member 101 in a manner which enables expansion (unwinding).

The reel 102 comprises two guide frames 103 which are provided in parallel and facing one another and with a separation distance of approximately the width of the base member 101, and a rolling core 104 which is provided between the central regions of the two guide frames 103 with the opposite ends of the core being connected to the two guide frames 103.

In order to ensure the access of liquids and light to the base member 101, the guide frames 103 are formed of the minimum framing necessary to enable the rolling of the base member 101 into a flat sheet.

For example, the guide frames 103 can comprise a hub 103a which connects to the rolling core 104, an outer ring 103b, and two spokes 103c for fixing the outer ring 103b to the hub 103a. The diameter of the outer ring 103b is larger than the external diameter of the rolled base member 101, while the diameter of the hub 103a is formed smaller than the diameter of the rolling core 104.

Moreover, it is also possible to provide one of the two guide frames 103 described above as detachable from the rolling core 104, and the conduct measurements in the integrated state, with the optical state measurements not obstructed by the spokes 103c.

Furthermore, it is also possible to identify those regions covered by the spokes 103c when the base member 101 is rolled about the aforementioned reel 102, and provide predetermined blank sections so that substances for detection are not bonded at those covered regions, with measurements then being conducted in the integrated state. Furthermore as shown in FIG. 9, it is also possible to measure the optical state in the expanded form, using a light emission section 39 and a light reception section 40.

In the figure, numeral 105 denotes a plurality of fixed locations provided along the length of the base member 101 at which a variety of substances for detection such as oligonucleotides are fixed. It is also possible to provide cavity sections such as channels, or holding sections comprising a porous material, a foam material, a fibrous material, or an impregnating material at the fixed locations. Moreover, the entire base member 101 can also be formed of a porous material, a foam material, a fibrous material, or an impregnating material.

In order to manufacture the integrated support 100, the tape shaped or cord shaped base member 101 is unwound from the reel 102 and reverted to the expanded state. In this state, a dispensing device is then used to dispense suspensions incorporating oligonucleotides with base sequences which correspond with the predetermined locations. The dispensing can be conducted with the aforementioned blank sections being avoided, so as to not effect the readings.

Next, with the base member still in the expanded state, the suspensions are dried and bonded to the base member 101.

Subsequently, the expanded base member 101 is rolled about the reel 102 by rotating the reel 102. Processing of the DNA fragment for base sequencing and the like is then conducted with the base member rolled around the reel 102.

To conduct a measurement of the optical state of an integrated support 100 for which processing has been carried out, either the opening provided by the guide frames 103 is used to carry out the measurement of the optical state of the integrated support 100 in the integrated state, or alternatively, the measurements are carried out either while the expanded support is being rolled on to the reel 102, or while the support is being unwound from the integrated state.

As follows is a description of a method of manufacturing an integrated support (a DNA integrated support) according to a seventh embodiment of the invention.

In a positioning step denoted step S1, a single film type or thin sheet type of base member is prepared. A plurality of channels are provided at predetermined locations on the base member in approximately parallel lines. Alternatively, a plurality of cavity sections such as capillaries could also be provided in parallel lines. Alternatively, a plurality of thread shaped, cord shaped, tape shaped, or rod shaped holding sections which are able to draw up or impregnate liquids and which comprise a porous material, a foam material, a fibrous material, or an impregnating material could also be provided in parallel lines.

In the case of cavity sections such as capillaries or holding sections, suspensions which have been drawn up or impregnated in advance are positioned by fixation so that suspensions which incorporate oligonucleotides with base sequences corresponding to the predetermined locations on the base member are positioned. In those cases where capillaries are used, capillaries are able to be brought in contact with one another, and so the substances for detection can be positioned with increased density. In contrast, in those cases where holding sections with impregnating ability are used, it is necessary to avoid any contact between holding sections.

In those cases where channels are provided on the base member, then suspensions incorporating oligonucleotides with base sequences corresponding to the fixed locations on the base member are dispensed into the channels, by using a dispensing device constructed with a pipette tip mounted to the aforementioned nozzle, and moving the dispensing device along the channels.

In the descriptions the term "line" is not necessarily restricted to straight lines, with curved lines also being possible. With curved lines, by consideration of the fact that as the film type base member is rolled the thickness of the base member will mean that the diameter from the center of the substrate will gradually increase, it is possible to gradually offset the channels to match this increase in diameter.

Furthermore in the positioning step of step S1 it is also possible to position the aforementioned suspensions in the channels by imprinting, using either an imprinting device wherein pressing enables the infusion of the suspensions and whereby using a printing surface which corresponds to the size and shape of the aforementioned lines the suspensions are positioned by imprinting into the channels, or alternatively an imprinting device wherein a stamp mount which has been soaked in, and which stores the suspension is used to press the suspension on to a printing surface which is then used to imprint the suspension on to the line.

In such cases, because a long line of the suspension is able to be positioned with one action, the positioning can be completed simply and rapidly. Furthermore, positioning can also be conducted by using a ball pen type tool in which the aforementioned suspension has been stored, and tracing along the line.

In addition, positioning can also be achieved by using a fountain pen type tool with a pen nib comprising a slit capable of retaining a liquid and in which the suspensions can be stored, or a pen type tool which will retain a suspension on immersion in a separate vessel containing the suspension, which is then used to trace out the aforementioned lines.

Next, at a bonding step denoted step S2, the aforementioned oligonucleotides suspended in the positioned suspensions are bonded to the base member, by drying the suspensions by heating for example.

Subsequently, at an integration step denoted step S3, the base member is integrated, in a manner which either enables or prevents expansion, by rolling in a direction which will not bend the channels provided in parallel lines, namely, in the direction perpendicular to the parallel lines so as to bring the surfaces of the base member together.

The regions enclosed by the channels and the surface of the base member form the cavity sections. An adhesive portion is provided on the surface as a binding section for binding the surfaces of the base member together in a manner which is either releasable or non-releasable. Consequently, by rolling the base member, the surfaces thereof are bonded together.

At a cutting step denoted step S4, the bound base member is cut in a direction approximately perpendicular to the aforementioned parallel lines, enabling the manufacture of a plurality of disk shaped integrated supports wherein the cut surface becomes the aforementioned layer surface.

Marking of the integrated medium can also be carried out by painting, mounting or the like and then fixing a predetermined light emitting substance such as a fluorescent material onto the parallel lines at predetermined locations during the positioning step of step S1.

As follows is a description of a method of manufacturing an integrated minute vessel according to an eighth embodiment of the invention.

When making integrated minute vessels, in contrast with the making of integrated supports, the positioning step of step S1 and the bonding step of step S2 are unnecessary.

However, a blocking step for blocking up one end of the aforementioned cavity sections is necessary following the integration step of step S3 and the cutting step of step S4. Furthermore in the case of the manufacture of a permeable membrane, of those steps outlined for the manufacture of integrated minute vessels, the blocking step is not required.

The embodiments above have been described in detail to further explain the present invention, but in no way preclude other embodiments. Consequently, the embodiments can be altered provided the gist of the invention is retained. For example, the above description of the embodiments refers to disk shaped DNA integrated supports, but the invention is not restricted to DNA, and can be used effectively with immune system substances, proteins, amino acids or sugars.

Furthermore, in the description above oligonucleotides were discussed as the substances for detection, but proteins, immune system substances, amino acids or sugars could also be used. Moreover, the description above focuses entirely on those cases where the system is expandable, but non-expandable systems are also possible.

Moreover in regards to reaction processing, a description was given of processing conducted on a single integrated support, but it is also possible to store multiple stages of integrated supports within the aforementioned dispensing device and conduct processing on the plurality of supports simultaneously.

Furthermore the base members may be either transparent or opaque. In the case of transparent base members, light can be captured not only from light emitting substances stored and fixed on the surfaces of the base members or cavity sections, but also from light emitting substances stored and fixed locations removed from the surface. Furthermore, it is also possible to form the base member from a conductive material, then integrate the base member by rolling, laminating or arranging while sandwiching a long and slender auxiliary member formed from an insulating material, and then passing a current along the length of the base member.

In such a case, the substances stored and fixed on the base member or in the cavity sections or holding sections are marked with electrochemical substances, and by passing an electrical current through the conductive base member, the light emitting materials will emit light which can then be measured.

## Claims

1. An integrated support comprising: at least one thread shaped, string shaped, tape shaped, or rod shaped long and slender base member, and a variety of substances for detection of predetermined chemical structure which are fixed side by side along the length of said base member, and said base member is rolled, laminated or arranged to give integration, so that a fixed location of each substance for detection corresponds to the chemical structure thereof.

2. An integrated support according to claim 1, wherein said base member is provided with cavity sections comprising a channel either with or without a bottom, an aperture, or a capillary, or holding sections of a porous material, a foam material, a fibrous material, a material with an irregular surface, or an impregnating material, and moreover wherein said substances for detection are fixed to said cavity sections or holding sections.

3. An integrated support according to either one of claim 1 and claim 2, wherein said base member is rolled, laminated or arranged in such a way that either enables or prevents expansion, while bringing side portions thereof into contact with each other or while maintaining a spacing or while sandwiching an auxiliary member.

4. An integrated support according to any one of claim 1 through claim 3, wherein markings are attached to said base member for identifying the chemical structure of said substances for detection, or the locations on said integrated support of said substances for detection.

5. An integrated support according to any one of claim 1 through claim 4, further comprising a binding section for binding said base member and/or an auxiliary member in such a way that is either releasable or non-releasable.

6. An integrated support according to claim 5, wherein said binding section is an adhesive portion for bonding side portions of said base member and/or auxiliary member in a manner which is either releasable or non-releasable.

7. An integrated support according to any one of claim 1 through claim 6, wherein a linear homoiothermal member is embedded inside said base member and/or an auxiliary member for heating or cooling purposes.

8. A DNA integrated support comprising:
a substantially flat substrate wherein at least one thread shaped, string shaped, tape shaped, or rod shaped long and slender base member is rolled, laminated or arranged in such a way that either enables or prevents expansion while bringing side portions thereof into contact with each other or while maintaining a spacing or while sandwiching an auxiliary member, to give integration; and
genetic substances such as oligonucleotides which are fixed in locations along the length of said base member, either in a plurality of cavity sections which comprise channels, apertures or capillaries either with or without bottoms, or alternatively in a plurality of holding sections comprising a porous material, a foam material, a fibrous material, a material with an irregular surface, or an impregnating material, with the cavity sections or holding sections being provided along the length of said base member,
and the fixed locations of each of said genetic substances correspond with base sequences for that substance.

9. An integrated minute vessel comprising: at least one thread shaped, cord shaped, tape shaped or rod shaped long and slender base member; and either cavity sections which comprise channels, apertures or capillaries with bottoms or ends, or holding sections having a porous material, a foam material, a fibrous material, a material with an irregular surface, or an impregnating material, provided on said base member, and wherein said base member is rolled, laminated or arranged to give integration.

10. An integrated minute vessel according to claim 9, wherein said base member is rolled, laminated or arranged in such a way that either enables or prevents expansion, while bringing side portions thereof into contact with each other or while sandwiching an auxiliary member, to give integration and formed into a substantially flat sheet.

11. An integrated minute vessel according to either one of claim 9 and claim 10, wherein marking is provided on a layer surface of the integrated minute vessel to identify position on the layer surface.

12. An integrated minute vessel according to any one of claim 9 through claim 11, wherein said integrated minute vessel is provided with a binding section for binding said base member and/or auxiliary member in such a way that is either releasable or non-releasable.

13. An integrated minute vessel according to any one of claim 9 through claim 12, wherein said binding section is an adhesive portion which bonds side portions of said base member and/or said auxiliary member in such a way that these are either releasable or non-releasable.

14. An integrated minute vessel according to any one of claim 9 through claim 13, wherein said thread shaped, cord shaped, tape shaped or rod shaped base member is provided with channels with ends, or apertures with bottoms or capillaries, and/or bottomless apertures or capillaries, or open ended channels, along the direction of a normal line of the layer surface.

15. An integrated minute vessel according to claim 9 through claim 14, wherein a linear homoiothermal member is provided inside said base member or said auxiliary member for heating or cooling purposes.

16. A permeable membrane comprising: at least one thread shaped, string shaped, tape shaped, or rod shaped long and slender base member; and cavity sections such as penetrating channels, apertures or capillaries, or holding sections formed from a porous material, a foam material, a fibrous material, a material with an irregular surface, or an impregnating material, provided on said base member, and wherein said base member is rolled, laminated or arranged to give integration.

17. A permeable membrane according to claim 16, wherein said base member is rolled, laminated or arranged in such a way that either enables or prevents expansion, while bringing side portions thereof into contact with each other or while sandwiching an auxiliary member to give integration, and formed into a substantially flat sheet.

18. A permeable membrane according to either one of claim 16 and claim 17, wherein said permeable membrane is provided with a binding section for binding said base member and said auxiliary member in such a way that is either releasable or non-releasable.

19. A permeable membrane according to claim 18, wherein said binding section is an adhesive portion which bonds side portions of said base member and/or said auxiliary member in such a way that these are either releasable or non-releasable.

20. A permeable membrane according to any one of claim 16 through claim 19, wherein said thread shaped, cord shaped, tape shaped or rod shaped long and slender base member is provided with cavity sections comprising open ended channels and/or bottomless apertures and/or capillaries along the direction of a normal line of the layer surface.

21. A permeable membrane according to any one of claim 16 through claim 20, wherein a linear homoiothermal member is provided inside said base member and/or said auxiliary member for heating or cooling purposes.

22. A method of manufacturing an integrated support, comprising a positioning step for positioning and fixing substances for detection of predetermined chemical structures at predetermined locations on at least one base member, and an integration step for rolling, laminating or arranging said base member to give integration, and the location of respective substances for detection and the chemical structures are made to correspond.

23. A method of manufacturing an integrated support according to claim 22, wherein said base member is formed from a thread shaped, string shaped, tape shaped, or rod shaped long and slender member.

24. A method of manufacturing an integrated support according to either one of claim 22 and claim 23, wherein in said positioning step, each suspension or semiliquid incorporating a substance for detection with a predetermined chemical structure, is positioned by being painted, dispensed, imprinted, drawn up, impregnated or stored onto said base member at a location which corresponds to the chemical structure.

25. A method of manufacturing an integrated support according to any one of claim 22 through claim 24, wherein in said integration step said base member is rolled, laminated or arranged in such a way that either enables or prevents expansion while bringing said base member into contact with itself or while maintaining a spacing or while sandwiching an auxiliary member to give integration.

26. A method of manufacturing an integrated support according to any one of claim 22, claim 24 and claim 25, wherein said base member is formed as a film or thin sheet, said substances for detection are positioned on said base member in approximate lines which do not intersect or contact the other substances, and said integration step involves rolling, laminating or arranging in a way that either enables or prevents expansion to give integration, and wherein a cutting step is provided following said integration step, in which the integrated base member on which said substances for detection are fixed, is sliced thinly to form a plurality of integrated supports in which the cross-sectional surface of the cut functions as a layer surface.

27. A method of manufacturing an integrated support according to any one of claim 22 through claim 26, wherein in said positioning step each suspension or semiliquid incorporating a substance for detection with a predetermined chemical structure, is positioned by being painted, dispensed, imprinted, drawn up, impregnated or stored onto said base member, or, into a plurality of cavity sections of channels, apertures or capillaries, or into holding sections having a porous material, a foam material, a fibrous material, a material with an irregular surface or an impregnating material, provided with said base member.

28. A method of manufacturing an integrated support according to either one of claim 22 and claim 27, wherein in said integrating step said base member and/or auxiliary member are bound in such a way that is either releasable or non-releasable.

29. A method of manufacturing an integrated support according to any one of claim 22 through claim 28, wherein in said positioning step, said substances for detection are fixed and supported onto said base member by drying the positioned suspensions or semiliquids which contain the substances for detection.

30. A method of manufacturing a DNA integrated support comprising:
a positioning step in which at each of a plurality of predetermined locations positioned along the length of at least one thread shaped, cord shaped, tape shaped or rod shaped long and slender base member, a suspension or semiliquid incorporating a genetic substance such as an oligonucleotide with a predetermined base sequence which corresponds to that particular location is positioned and fixed onto said base member by being painted, dispensed, imprinted, drawn up, impregnated or stored, either into cavity sections provided on said base member comprising channels, apertures or capillaries, or into holding sections provided on said base member having a porous material, a foam material, a fibrous material, a material with an irregular surface, or an impregnating material; and
an integration step wherein said base member, on which is positioned the suspensions or semiliquids, is rolled, laminated or arranged in such a way that either enables or prevents expansion, while bringing side portions of said base member into contact with each other or while maintaining a spacing or while sandwiching an auxiliary member to give integration.

31. A method of manufacturing a DNA integrated support comprising:
a positioning step in which at each of a plurality of predetermined locations side by side on at least one film type or thin sheet type base member, a suspension or semiliquid incorporating a genetic substance such as an oligonucleotide with a predetermined base sequence which corresponds to that particular location is positioned and fixed onto said base member in substantially parallel lines, into cavity sections comprising channels, apertures or capillaries and which are provided in substantially parallel lines on said base member, or into thread shaped, cord shaped, tape shaped or rod shaped long and slender holding sections which are provided in substantially parallel lines on said base member and which have a porous material, a foam material, a fibrous material, a material with an irregular surface, or an impregnating material by being painted, dispensed, imprinted, drawn up, impregnated or stored;
an integration step wherein said base member, on which is positioned the suspensions or semiliquids, is rolled, laminated or arranged in such a way that either enables or prevents expansion and so as not to bend said substantially parallel lines, while bringing face pairs of said base member into contact with each other or while maintaining a spacing or while sandwiching an auxiliary member, to give integration; and
a cutting step wherein said integrated base member is cut thinly across said substantially parallel lines to obtain a plurality of DNA integrated supports in which the cross-sectional surface of the cut functions as a layer surface.

32. A method of manufacturing an integrated minute vessel comprising:
a processing step for providing on at least one base member, either a plurality of cavity sections such as channels with bottoms or ends, or holding sections having a porous material, a foam material, a fibrous material, a material with an irregular surface, or an impregnating material; and
an integration step wherein said base member is rolled, laminated or arranged in such a way that either enables or prevents expansion, while bringing said base member into contact with itself or while maintaining a spacing or while sandwiching an auxiliary member, to give integration.

33. A method of manufacturing an integrated minute vessel according to claim 32, wherein said base member is formed as a film or thin sheet, and said cavity sections or said holding sections are each provided in approximate lines which do not intersect or contact the other sections, and wherein a cutting step for cutting the integrated base member to form a plurality of integrated minute vessels is provided after said integration step.

34. A method of manufacturing a permeable membrane comprising:
a processing step for providing on at least one base member, either a plurality of cavity sections such as bottomless or open-ended channels, or holding sections having a porous material, a foam material, a fibrous material, a material with an irregular surface, or an impregnating material; and
an integration step wherein said base member is rolled, laminated or arranged in such a way that either enables or prevents expansion, while bringing said base member into contact with itself or while maintaining a spacing or while sandwiching an auxiliary member to give integration..

35. A method of manufacturing a permeable membrane according to claim 34, wherein said base member is formed as film or thin sheet, and said cavity sections or said holding sections are each formed in approximate lines, and wherein a cutting step for cutting the integrated base member to form a plurality of permeable membranes is provided after said integration step.

36. A method of using an integrated medium, wherein by passing a heating fluid or a cooling fluid through an integrated support, an integrated minute vessel, or a permeable membrane according to any one of claim 1 through claim 21, the integrated support, integrated minute vessel, or permeable membrane is heated or cooled respectively.

37. A method of using an integrated medium, comprising:
a processing step for conducting processing using an integrated support, an integrated minute vessel, or a permeable membrane according to any one of claim 1 through claim 21, and
a measurement step for conducting measurements of an optical state with the processed integrated support, integrated minute vessel, or permeable membrane, either in an expanded state or in an integrated state.

38. A method of using an integrated medium according to claim 37, wherein the measurement in said measurement step with said integrated support, integrated minute vessel, or permeable membrane in an integrated state involves identification of an absolute location on the layer surface thereof.

39. A method of using an integrated minute vessel comprising:
a hardening step wherein with a base member of said integrated minute vessel according to any one of claim 1 through claim 21 in an expanded state, a predetermined suspension is positioned in a predetermined cavity section or a predetermined holding section by dispensing, imprinting, impregnating painting or storing, and subsequently hardened;
an integration step for integrating said base member on which said predetermined suspensions have been hardened, so that expansion is either enabled or prevented;
a fluidization step for fluidizing said hardened suspensions inside said respective cavity sections or holding sections; and
a processing step for conducting reaction processing within said cavity sections or holding sections.

40. A method of using an integrated minute vessel according to claim 39, wherein a suction step in which a pin shaped liquid passage is inserted into each of said cavity sections or holding sections for drawing up reaction products, is provided after said processing step.

41. A method of using an integrated minute vessel according to either one of claim 39 and claim 40, wherein said suspensions incorporate magnetic particles, and said suction step is conducted with a magnetic field either applied to, or absent from each of said cavity sections or holding sections.

42. An integrated medium storing fluid passage, comprising at least one fluid passage, and pressure control means for controlling the pressure inside said fluid passage, wherein an integrated support, an integrated minute vessel or a permeable membrane according to any one of claim 1 through claim 21 is stored inside said fluid passage, or inside a storage section communicated with said fluid passage, and said fluid is able to pass through the integrated support, integrated minute vessel or permeable membrane, or said fluid is able to contact the integrated support, integrated minute vessel or permeable membrane.

43. An integrated medium storing fluid passage according to claim 42, wherein said fluid passage, or said fluid passage and storage section are detachable with respect to said pressure control means, or said storage section or said integrated support, integrated minute vessel, or permeable membrane are detachable with respect to said fluid passage or storage section, and wherein said fluid passage and said pressure control means comprise displacement means for conducting relative displacement between vessels of said fluid passage, as well as a dispensing device.

44. An integrated medium storing fluid passage according to claim 42, wherein said pressure control means comprises a nozzle for controlling pressure by drawing gas from or discharging gas into said fluid passage, and said fluid passage comprises a reservoir section which stores fluid and which is joined in a detachable manner to said nozzle, and a narrow diameter section which is communicated with the reservoir section, and which is of smaller diameter than said reservoir section and is able to be inserted inside a vessel.

45. An integrated medium storing fluid passage according to any one of claim 42 through claim 44, wherein light emitting means for irradiating light on to said integrated support, integrated minute vessel, or permeable membrane stored in said fluid passage or said storage section, and/or light reception means for capturing light emitted from said integrated support, integrated minute vessel, or permeable membrane are provided.

46. An integrated medium storing fluid passage according to claim 45, further comprising a rotational drive section for rotational driving said fluid passage and said storage section, or said integrated support, integrated minute vessel, or permeable membrane contained therein.

47. An integrated medium storing fluid passage according to any one of claim 42 through claim 46, comprising a cooling medium or a heating medium provided outside of said fluid passage so as to be movable towards or away from an external wall of a neighboring fluid passage which contains said integrated medium, in order to heat or cool said integrated support, integrated minute vessel, or permeable membrane stored inside said fluid passage or inside said storage section communicated with said fluid passage.

48. An integrated medium storing fluid passage according to any one of claim 42 through claim 47, further comprising magnetic means which enables the application of or removal of a magnetic field acting in the direction of a normal line of said substrate, onto the inside of each of the cavity sections of said integrated support, integrated minute vessel, or permeable membrane stored inside said fluid passage or said storage section.

49. A magnetic separation device comprising: a mounting section for mounting an integrated support, integrated minute vessel, or permeable membrane according to any one of claim 1 through claim 21, and a magnetic section provided on a lower surface of said mounting section, and which enables the application of or removal of a magnetic field from beneath said substrate, with respect to the inside of each of said cavity sections of said integrated support, integrated minute vessel, or permeable membrane.
